# EUROPEAN PATENT SPECIFICATION

(11) **EP 4 322 910 B1**
(45) Date of publication and mention of the grant of the patent: **14.01.2026**
(21) Application number: 22722512.5
(22) Date of filing: 13.04.2022
(51) Int. Cl.: A61K 8/31, A61K 8/67, A61K 47/00, A23K 20/111, A23K 20/174, A23L 33/10, A23L 33/155, A61Q 19/02, A61Q 19/08

(54) **RETINOL OIL FORMULATION**
ÖLIGE RETINOLFORMULIERUNG
FORMULATION HUILEUSE DE RÉTINOL

(30) Priority: 16.04.2021 EP 21168786
(43) Date of publication of application: 21.02.2024
(73) Proprietor: DSM IP Assets B.V., 6221 BE Maastricht (NL)
(72) Inventor: HAENDEL, Linda, 4303 Kaiseraugst (CH); KOHLER, Lise, 4303 Kaiseraugst (CH); PORTA, Fabiola, 4303 Kaiseraugst (CH); SIEBER, Pascal, Christian, 4303 Kaiseraugst (CH); TSEKOU, Christos, 4303 Kaiseraugst (CH)
(74) Representative: dsm-firmenich IP
(86) International application number: PCT/EP2022/059823
(87) International publication number: WO 2022/219016

(56) References cited:
- EP-A1- 0 911 016
- CN-A- 111 544 415
- KR-A- 20200 051 997
- US-A- 5 851 538
- US-A1- 2017 042 801
- US-A1- 2020 085 703

## Description

The present invention relates to a new formulation, which comprises a high amount of retinol in a specific solvent and in the presence of mixed tocopherol.

Retinol, which is compound of the following formula 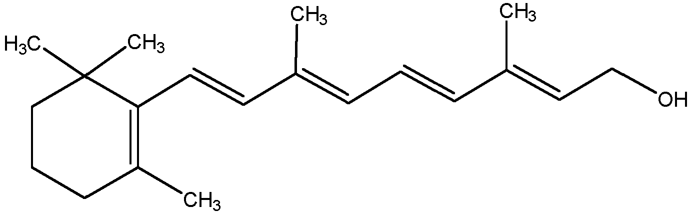 is a compound with very interesting properties in a variety of fields of applications.

Next to the application in food, feed and pharma, it is also very useful in personal care applications.

When used in the field of personal care it is mainly used for the maintenance of the skin.

Applying retinol topically it includes the following benefits:
- Preventing wrinkles due to its minimizing effect, as well as smoothing out existing fine lines and wrinkles.
- Brightening dull skin by exfoliating at a cellular level, which results in brighter and smoother new skin.
- Regulating oily skin and minimizing breakouts.
- Fading dark age spots, sun spots and hyperpigmentation and evening out complexion over time.

For the production of end-market products (like creams etc), it is necessary to provide a formulation comprising retinol, which can be incorporated into the end market product.

US2020/085703 describes a hair treatment product comprising a two-phase system, wherein the two phases comprise a water phase and an oil phase.

It is very advantageous that the formulation, which is used to produce the end-market product, comprises the retinol in a high amount, which means that not so much of solvent and other ingredients are present. This means that the concentration of such solvents and other ingredients in the end market product can kept low and the formulation of the present invention can be used in a wide range of applications.

Furthermore, such a formulation with a high amount of retinol needs to be stable so that the content of retinol is not decreasing during the storage of the formulation.

Furthermore, it was also a goal not to use antioxidants such as butylated hydroxytoluene (BHT) or butylated hydroxyanisole (BHA), because they are banned in a variety of countries for specific applications.

It was found that when a specific solvent (for the retinol) and mixed tocopherols (as antioxidant) were chosen then it was possible to produce a high concentrated and stable retinol formulation.

The choice of the specific solvent is crucial for the formulation according to the present invention. The solvent, wherein the retinol is solved must not comprise any alcohol group. The solvent which has been used is an apolar lipophilic hydrocarbon having no alcohol group and no ester group.

The present invention relates to a formulation (F) comprising
40 - 75 weight-% (wt-%), based on the total weight of the formulation, of retinol,
20 - 55 wt-%, based on the total weight of the formulation, of at least one solvent, and
0.1 - 5 wt-%, based on the total weight of the formulation, of mixed tocopherol,
wherein the at least one solvent is apolar lipophilic hydrocarbon having no alcohol group and no ester group,
wherein the formulation comprises less than 2 wt-%, based on the total weight of the formulation, of water.

The present invention relates to a formulation (F1) consisting of
40 - 75 wt-%, based on the total weight of the formulation, of retinol, and
20 - 55 wt-%, based on the total weight of the formulation, of at least one solvent, and
0.1 - 5 wt-%, based on the total weight of the formulation, of mixed tocopherol,
wherein the at least one solvent is apolar lipophilic hydrocarbon having no alcohol group and no ester group,
wherein the formulation comprises less than 2 wt-%, based on the total weight of the formulation, of water.

The present invention relates to a formulation (F2) consisting essentially of
40 - 75 wt-%, based on the total weight of the formulation, of retinol, and
20 - 55 wt-%, based on the total weight of the formulation, of at least one solvent, and
0.1 - 5 wt-%, based on the total weight of the formulation, of mixed tocopherol,
wherein the at least one solvent is apolar lipophilic hydrocarbon having no alcohol group and no ester group,
wherein the formulation comprises less than 2 wt-%, based on the total weight of the formulation, of water.

It is obvious that the percentages in all the formulations disclosed in the present patent application are always adding up to 100.

The formulation according to the present invention is no emulsion. The formulation according to the present invention is an oil formulation. This means that the water content of the inventive formulation can be kept as low as possible. No water is added to the formulation intentionally. It might be possible that the ingredients of the formulation according to the present invention can contain traces of water.

The oil formulation of retinol according to the present invention using the solvents of the present invention as solubilizer is ensuring an easy and more flexible use of such a solution in further applications, while an emulsification route (having more ingredients) of such active would have detrimental effect on final applications.

The present invention relates to a formulation (F'), which is formulation (F) wherein the formulation comprises less than 2 wt-%, based on the total weight of the formulation, of water.

The present invention relates to a formulation (F"), which is formulation (F) wherein the formulation comprises less than 1 wt-%, based on the total weight of the formulation, of water.

The present invention relates to a formulation (F‴), which is formulation (F) wherein the formulation comprises less than 0.5 wt-%, based on the total weight of the formulation, of water.

The retinol can be from a natural source or it can be produced chemically. Also mixture of such sourced retinols can be used. Some trace of other ingredient (impurities) can be present depending on its production or its extraction. But these impurities are usually present in an amount of less than 1 wt% (based on the weight of the retinol).

The amount of the retinol in the formulation according to the present invention is 40 - 75 wt-%, based on the total weight of the formulation.

Preferably the formulation according to the present invention comprises 40 - 70 wt-%, more preferably 42 - 70 wt-%, 42 - 65 wt-%, 45 - 65 wt-%, 45 - 60 wt-%, 45 - 55 wt-%, always based on the total weight of the formulation, of retinol.

Therefore, the present invention relates to a formulation (F3), which is formulation (F), (F'), (F"), (F‴), (F1) or (F2), comprising 40 - 70 wt-%, based on the total weight of the formulation, of retinol.

Therefore, the present invention relates to a formulation (F3'), which is formulation (F), (F'), (F"), (F‴), (F1) or (F2), comprising 42 - 70 wt-%, based on the total weight of the formulation, of retinol.

Therefore, the present invention relates to a formulation (F3"), which is formulation (F), (F'), (F"), (F‴), (F1) or (F2), comprising 42 - 65 wt-%, based on the total weight of the formulation, of retinol.

Therefore, the present invention relates to a formulation (F3‴), which is formulation (F), (F'), (F"), (F‴), (F1) or (F2), comprising 45 - 65 wt-%, based on the total weight of the formulation, of retinol.

Therefore, the present invention relates to a formulation (F3""), which is formulation (F), (F'), (F"), (F‴), (F1) or (F2), comprising 45 - 60 wt-%, always based on the total weight of the formulation, of retinol.

Therefore, the present invention relates to a formulation (F3ʺ‴), which is formulation (F), (F'), (F"), (F‴), (F1) or (F2), comprising 45 - 55 wt-%, based on the total weight of the formulation, of retinol.

As stated above the choice of the solvent is crucial for the stability of the formulation according to the present invention.

At least one apolar lipophilic hydrocarbon having no alcohol group and no ester group is used in the formulation according to the present invention.

Preferred solvents are alkanes, which can be linear or branched having 8 to 42 C atoms.

More preferred are C₁₀ - C₄₀ alkanes, which are linear or branched.

Especially preferred are the following solvents (as such or as mixtures) decane, undecane, dodecane, tridecane, tetradecane, pentadecane, hexadecane, heptadecane, octadecene, nonadecane, icosane, henicosane, docosane, tricosane, tetracosane, pentacosane, hexacosane, heptacosane, octacosane, nonacosane, triacontane, hentriacontane, dotriacontane, tritriacontane, tetratriacontane, pentatriacontane, hexatriacontane, heptatriacontane, octatriacontane, nonatriacontane and tetracontane. All of these alkanes can be linear as well as branched.

Most preferred are undecane, tridecane, pentadecane, hexadecane, heptadecane, octadecene, nonadecane and triacontane.

Such suitable solvents are available from a variety of suppliers (such as BASF, Seppic, and Aprinnova) under tradenames such as Emogreen L19, Emogreen L15, Cetiol Ultimate and Neossance Squalane.

Therefore, the present invention relates to a formulation (F4), which is formulation (F), (F'), (F"), (F‴), (F1), (F2), (F3), (F3'), (F3"), (F3‴), (F3ʺʺ) or (F3ʺ‴), wherein the at least one solvent is a C₈ - C₄₂ alkane, which can be linear or branched.

Therefore, the present invention relates to a formulation (F4'), which is formulation (F), (F'), (F"), (F‴), (F1), (F2), (F3), (F3'), (F3"), (F3‴), (F3"") or (F3ʺ‴), wherein the at least one solvent is a C₁₀ - C₄₀ alkane, which can be linear or branched.

Therefore, the present invention relates to a formulation (F4"), which is formulation (F), (F'), (F"), (F‴), (F1), (F2), (F3), (F3'), (F3"), (F3‴), (F3"") or (F3ʺ‴), wherein the at least one solvent is chosen from the group consisting of decane, undecane, dodecane, tridecane, tetradecane, pentadecane, hexadecane, heptadecane, octadecene, nonadecane, icosane, henicosane, docosane, tricosane, tetracosane, pentacosane, hexacosane, heptacosane, octacosane, nonacosane, triacontane, hentriacontane, dotriacontane, tritriacontane, tetratriacontane, pentatriacontane, hexatriacontane, heptatriacontane, octatriacontane, nonatriacontane and tetracontane, wherein these alkanes can be linear as well as branched.

Therefore, the present invention relates to a formulation (F4‴), which is formulation (F), (F'), (F"), (F‴), (F1), (F2), (F3), (F3'), (F3"), (F3‴), (F3"") or (F3ʺ‴), wherein the at least one solvent is chosen from the group consisting of undecane, tridecane, pentadecane, hexadecane, heptadecane, octadecene, nonadecane and triacontane, wherein these alkanes can be linear as well as branched.

The formulation according to the present invention comprises 20 - 55 wt-%, based on the total weight of the formulation, of at least one solvent.

Preferably 25 - 55 wt-%, 28- 55 wt-%, 35 -55 wt-%, always based on the total weight of the formulation, of at least one solvent.

Therefore, the present invention relates to a formulation (F5), which is formulation (F), (F'), (F"), (F‴), (F1), (F2), (F3), (F3'), (F3"), (F3‴), (F3ʺʺ), (F3ʺ‴), (F4), (F4'), (F4") or (F4‴), comprising 25 - 55 wt-%, based on the total weight of the formulation, of the at least one solvent.

Therefore, the present invention relates to a formulation (F5'), which is formulation (F), (F'), (F"), (F‴), (F1), (F2), (F3), (F3'), (F3"), (F3‴), (F3ʺʺ), (F3ʺ‴), (F4), (F4'), (F4") or (F4‴), comprising 28 - 55 wt-%, based on the total weight of the formulation, of the at least one solvent.

Therefore, the present invention relates to a formulation (F5"), which is formulation (F), (F'), (F"), (F‴), (F1), (F2), (F3), (F3'), (F3"), (F3‴), (F3ʺʺ), (F3ʺ‴), (F4), (F4'), (F4") or (F4‴), comprising 35 - 55 wt-%, based on the total weight of the formulation, of the at least one solvent.

The formulation according to the present invention comprises mixed tocopherols as antioxidant (0.1 - 5 wt-%, based on the total weight of the formulation).

Mixed tocopherol is a mixture of the following 4 compounds
α-tocopherol and
β-tocopherol and
γ-tocopherol and
δ-tocopherol.

Usually mixed tocopherol comprises
up to 20 wt-%, based on the total weight of the mixed tocopherol, of α-tocopherol and
up to 5 wt-%, based on the total weight of the mixed tocopherol, of β-tocopherol and
up to 75 wt-%, based on the total weight of the mixed tocopherol, of γ-tocopherol and
up to 35 wt-%, based on the total weight of the mixed tocopherol, of δ-tocopherol.

A preferred mixed tocopherol comprises
10 - 20 wt-%, based on the total weight of the mixed tocopherol, of α-tocopherol and
1 to 5 wt-%, based on the total weight of the mixed tocopherol, of β-tocopherol and
50 to 75 wt-%, based on the total weight of the mixed tocopherol, of γ-tocopherol and
15 to 35 wt-%, based on the total weight of the mixed tocopherol, of δ-tocopherol.

Therefore, the present invention relates to a formulation (F6), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5') or (F5"), wherein the mixed tocopherol comprises
up to 20 wt-%, based on the total weight of the mixed tocopherol, of α-tocopherol and
up to 5 wt-%, based on the total weight of the mixed tocopherol, of β-tocopherol and
up to 75 wt-%, based on the total weight of the mixed tocopherol, of γ-tocopherol and
up to 35 wt-%, based on the total weight of the mixed tocopherol, of δ-tocopherol.

Therefore, the present invention relates to a formulation (F6'), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5') or (F5"), wherein the mixed tocopherol comprises
10 - 20 wt-%, based on the total weight of the mixed tocopherol, of α-tocopherol and
1 to 5 wt-%, based on the total weight of the mixed tocopherol, of β-tocopherol and
50 to 75 wt-%, based on the total weight of the mixed tocopherol, of γ-tocopherol and
15 to 35 wt-%, based on the total weight of the mixed tocopherol, of δ-tocopherol.

Mixed tocopherols are commercially available from a variety of suppliers (such as BASF, DuPont, Merck and DSM).

The formulation according to the present invention does not comprise any further antioxidants (such as i.e. BHA and BHT) than the mixed tocopherol.

Therefore, the present invention relates to a formulation (F7), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6) or (F6'), wherein the formulation does not comprise any further antioxidants (other than the mixed tocopherol).

Therefore, the present invention relates to a formulation (F7'), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6) or (F6'), wherein the formulation is (essentially) free from BHA and BHT.

The formulations according to the present invention comprises 0.1 to 5 wt-%, based on the total weight of the present invention, of mixed tocopherol.

Preferably, the formulation according to the present invention comprises 0.2 to 4.5 wt-%, 0.2 to 4 wt-%, 0.3 to 4 wt-%, 0.4 to 3.5 wt-%, 0.4 to 3 wt-%, 0.4 to 2.5 wt-%, 0.4 to 2 wt-%, always based on the total weight of the formulation, of mixed tocopherol.

Therefore, the present invention relates to a formulation (F8), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7) or (F7'), comprising 0.2 - 4.5 wt-%, based on the total weight of the formulation, of mixed tocopherol.

Therefore, the present invention relates to a formulation (F8'), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7) or (F7'), comprising 0.2 - 4 wt-%, based on the total weight of the formulation, of mixed tocopherol.

Therefore, the present invention relates to a formulation (F8"), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7) or (F7'), comprising 0.3 - 4 wt-%, based on the total weight of the formulation, of mixed tocopherol.

Therefore, the present invention relates to a formulation (F8‴), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7) or (F7'), comprising 0.4 - 3.5 wt-%, based on the total weight of the formulation, of mixed tocopherol.

Therefore, the present invention relates to a formulation (F8""), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7) or (F7'), comprising 0.4 - 3 wt-%, based on the total weight of the formulation, of mixed tocopherol.

Therefore, the present invention relates to a formulation (F8ʺ‴), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7) or (F7'), comprising 0.4 - 2.5 wt-%, based on the total weight of the formulation, of mixed tocopherol.

Therefore, the present invention relates to a formulation (F8‴‴), which is formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7) or (F7'), comprising 0.4 - 2 wt-%, based on the total weight of the formulation, of mixed tocopherol.

The formulations according to the present invention are produced by using commonly known method and using commonly used devices.

A general way to produce the formulation according to the present invention is the following:
- Mixing the retinol and the mixed tocopherol at elevated temperature (usually at a temperature range of from 40 to 65°C)
- Heating the at least one solvent up to a similar temperature as the retinol/mixed tocopherol mixture (usually at a temperature range of from 40 to 65°C)
- Adding the at least one solvent to the retinol/mixed tocopherol mixture (or vice versa) at elevated temperature (usually at a temperature range of from 40 to 65°C) and mixing it at this temperature
- Cool down the mixture slowly.

Therefore, the present invention also relates to the process of producing any of the formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7), (F7'), (F8), (F8'), (F8"), (F8‴), (F8ʺʺ), (F8ʺ‴) or (F8‴‴) comprising the following steps
- Mixing the retinol and the mixed tocopherol at elevated temperature (usually at a temperature range of from 40 to 65°C)
- Heating the at least one solvent up to a similar temperature as the retinol/mixed tocopherol mixture (usually at a temperature range of from 40 to 65°C)
- Adding the at least one solvent to the retinol/mixed tocopherol mixture (or vice versa) at elevated temperature (usually at a temperature range of from 40 to 65°C) and mixing it at this temperature
- Cool down the mixture slowly.

It is also possible to mix the retinol in the solvent first (usually at a temperature range of from 40 to 65°C) and then mix it with the mixed tocopherol (usually at a temperature range of from 40 to 65°C) and the cool down the mixture slowly.

It is also possible to mix the mixed tocopherol in the solvent first (usually at a temperature range of from 40 to 65°C) and then mix it with the retinol (usually at a temperature range of from 40 to 65°C) and the cool down the mixture slowly.

The formulations according to the present invention can be used in a variety of fields of application, such as food, feed, pharma and personal care.

Preferably the formulations according to the present invention are used for incorporating into personal care products (such as creams, lotions, etc).

Therefore, the present invention also related to the use of at least one formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7), (F7'), (F8), (F8'), (F8"), (F8‴), (F8ʺʺ), (F8ʺ‴) or (F8‴‴) in food, feed, pharma and personal care products.

Therefore, the present invention also related to the use of at least one formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7), (F7'), (F8), (F8'), (F8"), (F8‴), (F8ʺʺ), (F8ʺ‴) or (F8‴‴) in personal care products (such as creams, lotions).

Furthermore, the present invention also relates to food, feed, pharma and personal care personal care products comprising at least one formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7), (F7'), (F8), (F8'), (F8"), (F8‴), (F8ʺʺ), (F8ʺ‴) or (F8‴‴).

Furthermore, the present invention also relates to personal care products (such as creams, lotions etc) comprising at least one formulation (F), (F'), (F"), (F‴), (F1), (F2), (F3), (F3'), (F3"), (F3‴), (F3ʺʺ), (F3ʺ‴), (F4), (F4'), (F4"), (F4‴), (F5), (F5'), (F5"), (F6), (F6'), (F7), (F7'), (F8), (F8'), (F8"), (F8‴), (F8ʺʺ), (F8ʺ‴) or (F8‴‴).

As stated above the one of the advantages of the formulation according to the present invention is the high amount of retinol (and therefore the reduced amount of other ingredients). Another very important advantage is that the formulation is in an oily form and not in form of a classical emulsion.

When incorporated into end-market products (food, feed, pharma and personal care personal care products) the amount of the formulation depends on how much retinol is needed in these final products.

The following examples serve to illustrate the invention.

### Examples

All the following examples are made according to the following method:
Melting and mixing the retinol and the mixed tocopherol a temperature of 63°C to 65°C for about 5 to 10min under nitrogen;
Then heating the solvent to a temperature of 63°C to 65°C.

Then mixing (under stirring) the retinol/mixed tocopherol mixture and the solvent at 63°C to 65°C for a few minutes 2 - 5 minutes and the let the formulation cool down to room temperature slowly.

The following solvents have been used are
Emogreen L19 (from Seppic): this is a C₁₅ - C₁₉ alkane mixture
Cetiol Ultimate (from BASF): this is a mixture of undecane and tridecane
Cetiol 5 (from BASF): this is a coco-caprylate (Comparison Example)
Eutanol G (from BASF): This is octyldodecanol (Comparison Example)

Mixed tocopherol was Mixed Tocopherols 95 (from DSM)

**Table1: All values in the table are wt-%, based on the total weight of the formulation**

| **Example** | **1** | **2** | **3** | **4** |
|---|---|---|---|---|
| Emogreen L19 | 51 | ---- | ---- | ---- |
| Eutanol G | | | | 51 |
| Cetiol Ultimate | ---- | 51.5 | ---- | ---- |
| Cetiol C5 | ---- | ---- | 51 | ---- |
| Retinol | 48 | 47.5 | 48 | 48 |
| Mixed tocopherol | 1 | 1 | 1 | 1 |

To determine the stability of these formulations, they were stored (at 40°C) for 2 weeks and 6 weeks. And the loss of retinol was measured (the initial value was 100%)

**Table 2: the retinol content was determined after storage**

| **Example** | **1** | **2** | **3** | **4** |
|---|---|---|---|---|
| **Initial** | 100 | 100 | 100 | 100 |
| **2 weeks** | 95.4 | 99.1 | 92.5 | 86.9 |
| **6 weeks** | 91 | 95 | 34 | 83.5 |

It can be seen from the table that the formulations according to the present invention are very stable and it can be seen how stability of the comparison examples 3 and 4 is lower than the one of the inventive formulation.

As stated above, the inventive retinol formulations of the present invention can be incorporated into a variety of compositions.

For example, in the following personal care compositions listed in the following tables (all values are given in weight-%, based on the total weight of the composition)

| **Examples: O/W-Emulsions** | **5** | **6** | **7** |
|---|---|---|---|
| Cetearyl Alcohol + Sodium Cetylstearylsulfat | 3.00 | 3.00 | 2.00 |
| Glycerylstearat SE | 2.00 | 2.00 | 4.00 |
| Octyldodecanol | 2.00 | 2.00 | 2.00 |
| C12-15 Alkylbenzoate | 1.00 | 1.00 | 1.00 |
| C13-16 Isoparaffin | 3.00 | 3.00 | 3.00 |
| Undecane, tridecane | 2.00 | 2.00 | 2.00 |
| Glycerine | 5.00 | 5.00 | 6.00 |
| Dimethicone | 0.50 | 0.50 | 0.50 |
| Sodium ascorbylphosphate | 0.10 | | |
| Ethylhexylsalicylate | 0.50 | 0.50 | 0.50 |
| Glycyrrhetic acid | - | - | 0.10 |
| Retinol formulation of Example 2 | 0.1 | 0.16 | 0.02 |
| BEL-EVEN^{™} | - | - | 0.50 |
| Grape seed oil | 0.50 | 0.50 | 0.50 |
| Dihydroxyacetone | - | - | 2.00 |
| Paraffinum Liquidum + Ginkgo Biloba Extract | 0.25 | 0.25 | 0.25 |
| Citric acid | 0.09 | 0.09 | 0.09 |
| Sodium citrate | 0.17 | 0.17 | 0.17 |
| Xanthan gum | - | - | 0.10 |

| | | | |
|---|---|---|---|
| Ammonium AcryloyldimethyltaurateNP Copolymer | 0.40 | 0.40 | - |
| Carbomer | - | - | 0.30 |
| Parabene | 0.30 | 0.30 | 0.30 |
| Phenoxyethanol | 0.50 | 0.50 | 0.50 |
| Alcohol Denat. | 3.50 | 3.50 | 3.50 |
| Perfume | q.s | q.s | q.s |
| Water | ad 100 | ad 100 | ad 100 |

| **Examples: Hydrodispersions** | **8** | **9** | **10** |
|---|---|---|---|
| Glyceryl Stearate Citrate | 0.4 | | |
| Sodium Carbomer | | | |
| Acrylates/C₁₀₋₃₀ Alkyl Acrylate Crosspolymer | | 0.1 | 0.2 |
| Ceteareth-20 | | | |
| Potassium Cetyl Phosphate | | | |
| Xanthan Gum | | 0.5 | 0.2 |
| Dimethicone/Vinyl Dimethicone Crosspolymer | | 3 | 1.5 |
| 2-(4-Diethylamino-2-hydroxybenzoyl)-benzoic Acid Hexylester | | 1.5 | |
| 2,4,6-Tribiphenyl-4-yl-1,3,5 Triazine | | | 0.5 |
| Butyl Methoxydibenzoylmethane | | | 5 |
| Ethylhexyl Bis-Isopentylbenzoxazolylphenyl Melamine | | 2 | |
| Bis-Ethylhexyloxyphenol Methoxyphenyltriazine | 2 | | |
| Disodium Phenyl Dibenzimidazole Tetrasulfonate | | 1 | |
| Phenylbenzimidazole Sulfonic Acid | 1 | | |
| Ethylhexyl Methoxycinnamate | | 8 | |
| Ethylhexyl Salicylate | | | |
| Homosalate | | | |
| Diethylhexyl Butamido Triazone | | | |
| Ethylhexyl Triazone | 3 | | 1 |
| Octocrylene | | | |
| Polysilicone-15 | 0.9 | | |
| Methylbenzylidene Camphor | | | 2 |
| BEL-EVEN^{™} | 0.5 | - | - |
| Titanium Dioxide | 2 | 1 | 2 |
| Drometrizole Trisiloxane | | 0.5 | |
| Terephthalidene Dicamphor Sulfonic Acid | | 0.75 | |
| C₁₂₋₁₅ Alkyl Benzoate | | | |
| Butylene Glycol Dicaprylate/Dicaprate | | | |
| Dicaprylyl Carbonate | 3 | | |
| Dicaprylylether | 2 | | |
| Cyclomethicone | | | 3 |
| 2-Phenylethylbenzoate | | | |
| Diethylhexylnaphthalate | | | |
| Tridecylsalicylate | 3 | 0.5 | 3 |
| PVP Hexadecene Copolymer | | 1 | |
| Glycerin | 5 | 8 | |
| Butylene Glycol | 7 | | |
| Glycine Soja | | | 1 |
| Vitamin E Acetate | 0.25 | 1 | 0.25 |
| Alpha-Glycosylrutin | | | |
| Retinol formulation of Example 2 | 0.1 | 0.02 | 0.1 |
| DHA | | 1.0 | |
| Erythrulose | | 0.5 | |
| Trisodium EDTA | 0.1 | | 0.1 |
| Tromethamine | | | |
| Ethanol | 10 | 1 | |
| Preservatives | q.s | q.s | q.s |
| Fragrance, Colours | q.s | q.s | q.s |
| Water | Ad 100 | Ad 100 | Ad 100 |

## Claims

1. A formulation comprising
40 - 75 weight-% (wt-%), based on the total weight of the formulation, of retinol,
20 - 55 wt-%, based on the total weight of the formulation, of at least one solvent, and
0.1 - 5 wt-%, based on the total weight of the formulation, of mixed tocopherol,
wherein the at least one solvent is apolar lipophilic hydrocarbon having no alcohol group and no ester group,
wherein the formulation comprises less than 2 wt-%, based on the total weight of the formulation, of water.

2. A formulation consisting (essentially) of
40 - 75 wt-%, based on the total weight of the formulation, of retinol, and
20 - 55 wt-%, based on the total weight of the formulation, of at least one solvent, and
0.1 - 5 wt-%, based on the total weight of the formulation, of mixed tocopherol,
wherein the at least one solvent is apolar lipophilic hydrocarbon having no alcohol group and no ester group,
wherein the formulation comprises less than 2 wt-%, based on the total weight of the formulation, of water.

3. The formulation according to claim 1 or claims 2, comprising 40 - 70 wt-%, based on the total weight of the formulation, of retinol.

4. The formulation according to any of the preceding claims, wherein the at least one solvent is a C₈ - C₄₂ alkane, which can be linear or branched.

5. The formulation according to any of the preceding claims, wherein the at least one solvent is chosen from the group consisting of decane, undecane, dodecane, tridecane, tetradecane, pentadecane, hexadecane, heptadecane, octadecene, nonadecane, icosane, henicosane, docosane, tricosane, tetracosane, pentacosane, hexacosane, heptacosane, octacosane, nonacosane, triacontane, hentriacontane, dotriacontane, tritriacontane, tetratriacontane, pentatriacontane, hexatriacontane, heptatriacontane, octatriacontane, nonatriacontane and tetracontane, wherein these alkanes can be linear as well as branched.

6. The formulation according to any of the preceding claims, comprising 25 - 55 wt-%, based on the total weight of the formulation, of the at least one solvent.

7. The formulation according to any of the preceding claims,
wherein the mixed tocopherol comprises
up to 20 wt-%, based on the total weight of the mixed tocopherol, of α-tocopherol and
up to 5 wt-%, based on the total weight of the mixed tocopherol, of β-tocopherol and
up to 75 wt-%, based on the total weight of the mixed tocopherol, of γ-tocopherol and
up to 35 wt-%, based on the total weight of the mixed tocopherol, of δ-tocopherol.

8. The formulation according to any of the preceding claims, wherein the formulation does not comprise any further antioxidants (other than the mixed tocopherol).

9. The formulation according to any of the preceding claims, comprising 0.2 - 4.5 wt-%, based on the total weight of the formulation, of mixed tocopherol.

10. Process of producing a formulation according to any of claims 1 - 9 comprising the following steps
• Mixing the retinol and the mixed tocopherol at elevated temperature (40 to 65°C)
• Heating the at least one solvent up to a similar temperature as the retinol/mixed tocopherol mixture (40 to 65°C)
• Adding the at least one solvent to the retinol/mixed tocopherol mixture (or vice versa) at elevated temperature (40 to 65°C) and mixing it at this temperature
• Cool down the mixture slowly.

11. Use of at least one formulation according to any of claims 1 - 9 in food, feed, pharma and personal care products (preferably in personal care products).

12. Food, feed, pharma and personal care personal care products comprising at least one formulation according to any of claims 1 - 9.

13. Personal care personal care products comprising at least one formulation according to any of claims 1 - 9.

## Patentansprüche

1. Formulierung, umfassend
40 - 75 Gewichtsprozent (Gew.-%), bezogen auf das Gesamtgewicht der Formulierung, Retinol,
20 - 55 Gew.-%, bezogen auf das Gesamtgewicht der Formulierung, wenigstens eines Lösungsmittels und
0,1 - 5 Gew.-%, bezogen auf das Gesamtgewicht der Formulierung, gemischtes Tocopherol,
wobei das mindestens eine Lösungsmittel unpolarer lipophiler Kohlenwasserstoff ist, der keine Alkoholgruppe und keine Estergruppe aufweist,
wobei die Formulierung weniger als 2 Gew.-%, bezogen auf das Gesamtgewicht der Formulierung, Wasser umfasst.

2. Formulierung, (im Wesentlichen) bestehend aus 40 - 75 Gew.-%, bezogen auf das Gesamtgewicht der Formulierung, Retinol und
20 - 55 Gew.-%, bezogen auf das Gesamtgewicht der Formulierung, wenigstens eines Lösungsmittels und
0,1 - 5 Gew.-%, bezogen auf das Gesamtgewicht der Formulierung, gemischtes Tocopherol,
wobei das mindestens eine Lösungsmittel unpolarer lipophiler Kohlenwasserstoff ist, der keine Alkoholgruppe und keine Estergruppe aufweist,
wobei die Formulierung weniger als 2 Gew.-%, bezogen auf das Gesamtgewicht der Formulierung, Wasser umfasst.

3. Formulierung nach Anspruch 1 oder Anspruch 2, umfassend 40 - 70 Gew.-%, bezogen auf das Gesamtgewicht der Formulierung, Retinol.

4. Formulierung nach einem der vorhergehenden Ansprüche, wobei das mindestens eine Lösungsmittel ein C₈-C₄₂-Alkan ist, das geradkettig oder verzweigt sein kann.

5. Formulierung nach einem der vorhergehenden Ansprüche, wobei das mindestens eine Lösungsmittel aus der aus Decan, Undecan, Dodecan, Tridecan, Tetradecan, Pentadecan, Hexadecan, Heptadecan, Octadecen, Nonadecan, Icosan, Henicosan, Docosan, Tricosan, Tetracosan, Pentacosan, Hexacosan, Heptacosan, Octacosan, Nonacosan, Triacontan, Hentriacontan, Dotriacontan, Tritriacontan, Tetratriacontan, Pentatriacontan, Hexatriacontan, Heptatriacontan, Octatriacontan, Nonatriacontan und Tetracontan bestehenden Gruppe ausgewählt ist, wobei diese Alkane sowohl geradkettig als auch verzweigt sein können.

6. Formulierung nach einem der vorhergehenden Ansprüche, umfassend 25 - 55 Gew.-%, bezogen auf das Gesamtgewicht der Formulierung, des mindestens einen Lösungsmittels.

7. Formulierung nach einem der vorhergehenden Ansprüche,
wobei das gemischte Tocopherol Folgendes umfasst
bis zu 20 Gew.-%, bezogen auf das Gesamtgewicht des gemischten Tocopherols, α-Tocopherol und
bis zu 5 Gew.-%, bezogen auf das Gesamtgewicht des gemischten Tocopherols, β-Tocopherol und
bis zu 75 Gew.-%, bezogen auf das Gesamtgewicht des gemischten Tocopherols, γ-Tocopherol und
bis zu 35 Gew.-%, bezogen auf das Gesamtgewicht des gemischten Tocopherols, δ-Tocopherol.

8. Formulierung nach einem der vorhergehenden Ansprüche, wobei die Formulierung keine weiteren Antioxidantien (außer dem gemischten Tocopherol) umfasst.

9. Formulierung nach einem der vorhergehenden Ansprüche, umfassend 0,2 - 4,5 Gew.-%, bezogen auf das Gesamtgewicht der Formulierung, gemischtes Tocopherol.

10. Verfahren zur Herstellung einer Formulierung nach einem der Ansprüche 1 - 9, welches die folgenden Schritte umfasst
• das Mischen des Retinols und des gemischten Tocopherols bei erhöhter Temperatur (40 bis 65 °C)
• das Erwärmen des mindestens einen Lösungsmittels auf eine ähnliche Temperatur wie die Mischung aus Retinol/gemischtem Tocopherol (40 bis 65 °C)
• die Zugabe des mindestens einen Lösungsmittels zu der Mischung aus Retinol/gemischtem Tocopherol (oder umgekehrt) bei erhöhter Temperatur (40 bis 65 °C) und das Mischen bei dieser Temperatur
• das langsame Abkühlen der Mischung.

11. Verwendung mindestens einer Formulierung nach einem der Ansprüche 1 - 9 in Lebens-, Futter-, Pharma- und Körperpflegeprodukten (vorzugsweise in Körperpflegeprodukten).

12. Lebensmittel-, Futter-, Pharma- und Körperpflegeprodukte, die mindestens eine Formulierung nach einem der Ansprüche 1 bis 9 umfassen.

13. Körperpflegeprodukte, die mindestens eine Formulierung nach einem der Ansprüche 1 - 9 umfassen.

## Revendications

1. Formulation comprenant
40 à 75 % en poids (% en poids), par rapport au poids total de la formulation, de rétinol,
20 à 55 % en poids, par rapport au poids total de la formulation, d'au moins un solvant, et
0,1 à 5 % en poids, par rapport au poids total de la formulation, de tocophérol mixte,
dans laquelle l'au moins un solvant est un hydrocarbure lipophile apolaire n'ayant pas de groupe alcool et pas de groupe ester,
dans laquelle la formulation comprend moins de 2 % en poids, par rapport au poids total de la formulation, d'eau.

2. Formulation constituée (essentiellement) de 40 à 75 % en poids, par rapport au poids total de la formulation, de rétinol, et
20 à 55 % en poids, par rapport au poids total de la formulation, d'au moins un solvant, et
0,1 à 5 % en poids, par rapport au poids total de la formulation, de tocophérol mixte,
dans laquelle l'au moins un solvant est un hydrocarbure lipophile apolaire n'ayant pas de groupe alcool et pas de groupe ester,
dans laquelle la formulation comprend moins de 2 % en poids, par rapport au poids total de la formulation, d'eau.

3. Formulation selon la revendication 1 ou la revendication 2, comprenant 40 à 70 % en poids, par rapport au poids total de la formulation, de rétinol.

4. Formulation selon l'une quelconque des revendications précédentes, dans laquelle l'au moins un solvant est un alcane en C₈ - C₄₂, qui peut être linéaire ou ramifié.

5. Formulation selon l'une quelconque des revendications précédentes, dans laquelle l'au moins un solvant est choisi dans le groupe constitué de décane, undécane, dodécane, tridécane, tétradécane, pentadécane, hexadécane, heptadécane, octadécène, nonadécane, icosane, hénicosane, docosane, tricosane, tétracosane, pentacosane, hexacosane, heptacosane, octacosane, nonacosane, triacontane, hentriacontane, dotriacontane, tritriacontane, tétratriacontane, pentatriacontane, hexatriacontane, heptatriacontane, octatriacontane, nonatriacontane et tétracontane, dans laquelle ces alcanes peuvent être linéaires ou ramifiés.

6. Formulation selon l'une quelconque des revendications précédentes, comprenant 25 à 55 % en poids, par rapport au poids total de la formulation, de l'au moins un solvant.

7. Formulation selon l'une quelconque des revendications précédentes,
dans laquelle le tocophérol mixte comprend jusqu'à 20 % en poids, par rapport au poids total du tocophérol mixte, de α-tocophérol et
jusqu'à 5 % en poids, par rapport au poids total du tocophérol mixte, de β-tocophérol et
jusqu'à 75 % en poids, par rapport au poids total du tocophérol mixte, de γ-tocophérol et
jusqu'à 35 % en poids, par rapport au poids total du tocophérol mixte, de δ-tocophérol.

8. Formulation selon l'une quelconque des revendications précédentes, dans laquelle la formulation ne comprend pas d'autres antioxydants (autres que le tocophérol mixte).

9. Formulation selon l'une quelconque des revendications précédentes, comprenant 0,2 à 4,5 % en poids, par rapport au poids total de la formulation, de tocophérol mixte.

10. Procédé de production d'une formulation selon l'une quelconque des revendications 1 à 9, comprenant les étapes suivantes
• mélange du rétinol et du tocophérol mixte à température élevée (40 à 65 °C)
• chauffage de l'au moins un solvant jusqu'à une température similaire à celle du mélange rétinol/tocophérol mixte (40 à 65 °C)
• ajout de l'au moins un solvant dans le mélange rétinol/tocophérol mixte (ou vice versa) à température élevée (40 à 65 °C) et mélange de celui-ci à cette température
• refroidissement du mélange lentement.

11. Utilisation d'au moins une formulation selon l'une quelconque des revendications 1 à 9 dans des produits alimentaires, des produits alimentaires pour animaux, des produits pharmaceutiques et des produits de soins personnels (de préférence dans des produits de soins personnels).

12. Produits alimentaires, produits alimentaires pour animaux, produits pharmaceutiques et produits de soins personnels comprenant au moins une formulation selon l'une quelconque des revendications 1 à 9.

13. Produits de soins personnels comprenant au moins une formulation selon l'une quelconque des revendications 1 à 9.
